# EUROPEAN PATENT SPECIFICATION

(11) **EP 1 177 184 B1**
(45) Date of publication and mention of the grant of the patent: **25.08.2004**
(21) Application number: 00930265.4
(22) Date of filing: 01.05.2000
(51) Int. Cl.: C07D 303/48, C07D 303/08, C07D 301/26, C07D 301/03, C08G 65/00, C08F 16/26, C08G 65/22

(54) **POLYFLUORINATED EPOXIDES AND ASSOCIATED POLYMERS AND PROCESSES**
POLYFLUORIERTE EPOXIDE UND ASSOZIERTE POLYMERE UND VERFAHREN
EPOXYDES POLYFLUOREES, POLYMERES ASSOCIES ET PROCEDES

(30) Priority: 04.05.1999 US 132453 P
(43) Date of publication of application: 06.02.2002
(73) Proprietor: E.I. DU PONT DE NEMOURS AND COMPANY, Wilmington, Delaware 19898 (US)
(72) Inventor: PETROV, Viacheslav, Alexandrovich, Hockessin, DE 19707 (US); FEIRING, Andrew, Edward, Wilmington, DE 19807 (US); FELDMAN, Jerald, Hockessin, DE 19707 (US)
(74) Representative: Towler, Philip Dean
(86) International application number: PCT/US2000/011746
(87) International publication number: WO 2000/066575

(56) References cited:
- EP-A- 0 064 293
- EP-A- 0 100 488
- EP-A- 0 414 569
- EP-A- 0 473 398
- EP-A- 0 889 028
- WO-A-00/17712
- WO-A-00/67072
- US-A- 3 275 573
- US-A- 3 573 330
- US-A- 5 084 583
- US-A- 5 808 132
- SIMMONS H. E.: "Fluoroketones" JOURNAL OF THE AMERICAN CHEMICAL SOCIETY., vol. 82, no. 9, 1960, pages 2288-2296, XP002145136 AMERICAN CHEMICAL SOCIETY, WASHINGTON, DC., US ISSN: 0002-7863
- BRYCE M. R.: "Reactions involving fluoride ion. Part 30. Preparation and reactions of epoxides derived from perfluoroalkyl substituted alkenes" JOURNAL OF THE CHEMICAL SOCIETY, PERKIN TRANSACTIONS 1., no. 7, 1984, pages 1391-1395, XP002145137 CHEMICAL SOCIETY. LETCHWORTH., GB ISSN: 1470-4358
- GOLUBEV A. S.: "Cycloaddition reactions of the methyl ester of trifluoropyruvic acid" BULLETIN OF THE ACADEMY OF SCIENCES OF THE USSR. DIVISION OF CHEMICAL SCIENCE., vol. 37, no. 1, - 20 July 1988 (1988-07-20) pages 117-121, XP002145138 CONSULTANTS BUREAU. NEW YORK., US
- DATABASE WPI Section Ch, Week 199425 Derwent Publications Ltd., London, GB; Class A25, AN 1994-206550 XP002154159 & JP 06 145338 A (JAPAN ENERGY KK), 24 May 1994 (1994-05-24)

## Description

This invention is in the field of polyfluorinated epoxides and includes methods for producing these epoxides, certain chemical reactions these epoxides undergo, and monomers and polymers derived from these epoxides and/or their derivatives.

Various polyfluorinated epoxides are known. As an illustrative example, 1,1-bis(trifluoromethyl)ethylene oxide is known and can be produced by reaction of diazomethane and hexafluoroacetone: see Chang, I. S., Willis, C. J., Can. J. Chem. 1997, 55, 2465. While this production method can be done on a lab scale, it does involve use of hazardous diazomethane and is not capable of being scaled up for producing larger quantities. As a second illustrative example, the compound illustrated below is also known. 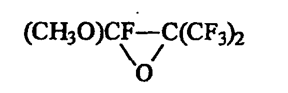 The production of this compound, again involving a hazardous reagent, has been reported via oxidation of the corresponding olefin with ozone. See JP Patent Publication 08333302 A2. Neither of the aforementioned production processes is attractive for commercial production for the reasons presented above.

The preparation of perfluorinated or perhalogenated epoxides using sodium hypochlorite or sodium hypobromide is known. See: Kolenko, I. P., Filaykova, T. I., Zapevalov, A. Yu., Lur'e, E. P. *Izv. AN USSR Ser. Khim. 1979,* p. 2509; and Coe, P. L., Mott, A. W., Tatlow, J. C., *J. Fluorine Chemistry, 1985,* V30, p. 297.

EP-A-100488 discloses a process for epoxidation of a fluoro-olefin of the general formula (X¹)(X²)C=C(X³)(CF₂Y¹) wherein X¹, X² and X³ each represents a substituent selected from (a) -F, (b) a perfluoroalkyl group having 2 to 20 carbon atoms and (c) -CF₂Y¹, Y¹ may be the same or different and represents a substituent selected from (d) a halogen atom selected from F, Cl, Br and I, (e) -OZ¹ and (f) -Z¹, wherein Z¹ may be the same or different and represents a substituted or unsubstituted hydrocarbon group having not more than 20 carbon atoms, and X¹, X², X³ and Y¹ may be combined with one another to form a cyclic compound, provided that all of X¹, X², X³ and Y¹ do not represent -F; using a hypochlorite dissolved or suspended in an aqueous phase as an oxidizing agent in the presence or absence of an inorganic base. The reaction is performed in a two phase system (aqueous and organic phases) in the presence of at least one phase transfer catalyst selected from (i) quaternary ammonium salts, (ii) quaternary phosphonium salts, (iii) quaternary arsonium salts, (iv) sulfonium salts and (v) lipophilic complexing agents for cations contained in the hypochlorite.

Bryce, M.R., Chambers, RD. and Kirk,J.R, *J. Chem. Soc. Perkin Trans. I,* 1984, p.1391 describe the preparation of epoxides derived from perfluoroalkyl substituted alkenes. The stability of these epoxides is attributed to the presence of bulky, electronegative perfluoroalkyl groups which both hinder attack by nucleophiles and also withdraw electrons from the oxiran ring.

There is a need for a safe and efficient production method for producing polyfluorinated epoxide compounds as well as safe efficient processes for converting them into useful polymeric products.

### SUMMARY OF THE INVENTION

In one embodiment, the invention is a method for producing a fluorinated epoxide in high yield, said method comprising the step of reacting a fluorinated ethylenically unsaturated compound having the structure:

(R₁)(R₂)C=C(R₃)(R₄)

with a metal hypohalite oxidizing agent in the presence of a phase transfer catalyst to produce the fluorinated epoxide having the structure: 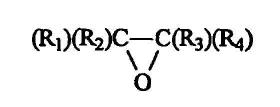 wherein R₁ is selected from the group consisting of H and OR, where R is C₁-C₁₀ alkyl; R₂ is selected from the group consisting of H, F, C₁-C₁₀ perfluoroalkyl, and X-substituted C₁-C₁₀ alkyl, wherein X is F, Cl, Br, I, OH, or OR; R₃ and R₄ are each independently selected from the group consisting of C₁-C₁₀ perfluoroalkyl, C(R_{f})(R_{f}')OH where R_{f} and R_{f}' are C₁-C₁₀ perfluoroalkyl groups, C₁-C₁₀ perfluoroalkoxy, C₁-C₁₀ carboalkoxy, and hydroxy-substituted C₁-C₁₀ carboalkoxymethyl-substituted C₁-C₄ perfluoroalkyl.

Preferably, R₁ is selected from the group consisting of H and OR, where R is C₁-C₁₀ alkyl; R₂ is selected from the group consisting of H and F; and R₃ and R₄ are selected from the group consisting of C₁-C₁₀ perfluoroalkyl and C₁-C₁₀ perfluoroalkoxy.

### DETAILED DESCRIPTION OF THE PREFERRED EMBODIMENT(S)

In one embodiment, the invention is a method for producing a fluorinated epoxide comprising the step of reacting a fluorinated ethylenically unsaturated compound (structure given supra) with a metal hypohalite oxidizing agent in the presence of a phase transfer catalyst to produce the fluorinated epoxide (structure also given supra).

A suitable oxidizing agent is a metal hypohalite. Exemplary metal hypohalites include, but are not limited to, various metal hypochlorites or metal hypobromites, including lithium, sodium, potassium, and calcium hypochlorites or hypobromites. Preferred oxidizing agents are sodium, calcium, or potassium hypochlorite and sodium or potassium hypobromite.

Examples of suitable phase-transfer catalysts include, but are not limited to, tetraethylammonium chloride, tetraethylammonium bromide, tetramethylammonium hydroxide, tetrabutylammonium chloride, tetrabutylammonium bromide, tetrabutylammonium hydroxide, trimethylbenzylammonium chloride, trimethylbenzylammonium bromide, methyltricaprylyl halide, methyltricaprylyl hydroxide, and trimethylbenzylammonium hydroxide. Preferred are methyltricaprylyl halide or hydroxide.

In another embodiment, the invention is a method for producing a polyfluorinated polyether comprising the step of reacting a fluorinated epoxide having the structure given supra with a basic compound in solvent or neat to produce the polyfluorinated polyether as given supra. Suitable bases for use in this reaction include, but are not limited to, trialkylamines of formula R₁R₂R₃N, where R₁-R₃ are independently C₁-C₆ alkyl; pyridine, sodium or potassium alkoxides (e.g., methoxide, ethoxide, t-butoxide), and sodium or potassium hydroxide.

The polyfluorinated epoxides of this invention having the structure: 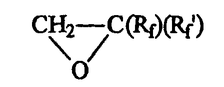 can be reacted with a variety of compounds (as illustrated in Examples 3 -6, 8) to afford new compounds comprised of the structure:

### - X-CH₂C(R_{f})R_{f}')OH

### GLOSSARY

Term "F-" in a compound name designates that the compound is perfluorinated.

### EXAMPLES

### EXAMPLE 1

### Preparation of 1,1-Bis(trifluoromethyl)ethylene Oxide (1)

Hexafluoroisobutene CH₂=C(CF₃)₂ (25 ml, 40 g) was condensed in a flask containing a solution of NaOCl (made at -5 to -3°C by bubbling 15 g of chlorine into mixture of 50 ml of 50 wt. % of NaOH and 100 ml of water) and 0.5 g of phase transfer catalyst - methyltricaprylylammonium chloride (Aliquat™ -336, Aldrich) was added at -2 to +2°C under vigorous stirring. Reaction mixture was agitated at this temperature for 1-1.5 hours.

The resulting reaction product was transferred out of the reactor in vacuum, collected in a cold trap (at-78°C) and distilled to give 37.5 g (yield 86%) of liquid, b.p. 41-42°C/760 mm Hg, which was identified as 1,1-bis(trifluoromethyl)ethylene oxide (1). The resulting compound 1 was established to have the indicated structure based upon the analytical data obtained as indicated below.
¹H NMR: 3.28 (s) ppm
¹⁹F NMR: -73.34 (s) ppm
¹³C {H} NMR: 46.75 (s), 54.99 (sept, 37 Hz), 126.76 (q, 275 Hz)
IR (gas, major): 1404 (s), 1388 (s), 1220 (s), 1083 (s), 997 (m), 871 (m), 758 (w), 690 (m), 636 (w) cm⁻¹
Anal. Calcd for C₄H₂F₆O: C, 26.68, H1.12. Found: C, 27.64, H, 1.10

### EXAMPLE 2

### Preparation of 1-Methoxy-F-2,2-Dimethylethylene Oxide

Olefin CH₃OCF=C(CF₃)₂ (4.8 g) was added dropwise into a flask containing a solution of NaOCl (made at -5 to -3°C by addition of 2.5 ml of chlorine into solution of 10 ml 50% NaOH in 20 ml of water) and 0.3 g of phase transfer catalyst - methyl tricaprylyl ammonium chloride (Aliquat™-336, Aldrich) at -2 to 0°C under vigorous stirring. Reaction mixture was kept at this temperature for 40 min, diluted with water, organic layer (lower) was separated, washed with water, dried over MgSO₄ and analyzed. Based on NMR data crude product was found to be a mixture of 80% of 1-methoxy-*F*-2,2-dimethylethylene oxide and 20% of (CF₃)₂CClC(O)OCH₃.
Epoxide: ¹H NMR: 3.53 (d, 1 Hz) ppm.
¹⁹F NMR: -69.06 (3F, dt, 8; 19 HZ), -64.40 (3F, qd, 8; 1 Hz), -110.90 (1F, m) ppm.
IR: 1490 (s, epoxide), cm⁻¹.

### COMPARATIVE EXAMPLE 1

### Attempt of Oxidation of C₄F₉CH=CH₂

In an attempted reaction of 5 g of C₄F₉CH=CH₂ with solution of NaOCl (made out of 10 ml f 50% NaOH, 20 ml of H₂O, 3 ml of chlorine) and 0.3 g of phase transfer catalyst - methyl tricaprylyl ammonium chloride (Aliquat™ -336, Aldrich) at -2 to 0°C for 2 h only starting fluoroolefin was recovered and no detectable reaction products were found.

### EXAMPLE 3

### Synthesis of 1,1-Bis{trifluoromethyl)-2-Chloromethyl Oxirane

Using 100 ml of NaOCl (Aldrich, 12% chlorine available), 0.5 g of phase transfer catalyst, tricaprylylmethylammonium chloride (Aliquat™ -336, Aldrich) and 28 g of (CF₃)₂C=CHCH₂Cl, which is added slowly at 5-15°C, after stirring reaction mixture for 1h at 15-20°C and separation of layer, there is isolated 26 g of crude product, containing 84% epoxide and 16% of (CF₃) ₂CHCH=CHCl (NMR). Distillation of crude material using short spinning-band column give 7 g (78% calculated and 25% isolated yield respectively) of epoxide 98% purity, b.p. 88.2-88.6°C. NMR: ¹H (acetone-d₆): 4.12 (1H, m), 3.95 (1Hm). 4.20(1H,m); ¹⁹F: 74.11 (3F, q; 7Hz), -67.21(3F, q;7Hz); ¹³C (proton decoupled) 34.42(q; 4Hz), 58.94(q; 3Hz), 59.53(sept.; 40Hz), 120.37(q. ; 281Hz), 120.96(q.; 281Hz). IR: 1459 cm⁻¹.

### EXAMPLE 4

### Synthesis of 1,1-Bis(Perfluoroethyl)-2-n-Perfluoropropyl Oxirane

Using 18 ml of NaOCl (Aldrich, 12% chlorine available), 0.2 g of phase transfer catalyst (Aliquat™ -336, Aldrich, tricaprylylmethylammonium chloride) and 3 g of (C₂F₅)₂C=CHC₃F₇, which is added slowly at 5-15°C, after stirring reaction mixture at 15-20°C for 15 h, separation of lower layer it is isolated 2.5 g of crude product, which is based on NMR data epoxide of >98% purity. Yield is 83%. IR: 1354; 1353 cm⁻¹; ¹H NMR
(CDCl₃): 3.85 (d.d); ¹⁹F NMR: -81.55 (3F, d), -80.92(3F, t), -81.10 (3F, t), -11.50 (2F, AB pattern), -119.00 (2F, AB pattern), -119.80 (2F, AB pattern), -127.80 (2F, AB pattern).

### EXAMPLE 5

### Synthesis of CH₂(O)C[C(CF₃)₂OH]C(O)OCH₃ (3)

Using 30 ml of NaOCl (Aldrich, 12% chlorine available), 0.2 g of phase transfer catalyst (Aliquat-336, Aldrich, tricaprylylmethylammonium chloride) and 7 g of CH₂=C[C(CF₃)₂O]C(O)OCH₃, which is added slowly at 5-15°C, after stirring reaction mixture at 15-20°C for 1.5 h, reaction mixture is filtered. There is isolated 4.0 g of product which, based on NMR data, is epoxide 3 of >98% purity, m.p. 56-58°C. Yield is 57%. IR: 1744; 1450 cm⁻¹; ¹H NMR (CDCl₃): 3.15 (1H, d, 5.6Hz) 3.25 (1H, d, 5.6Hz); 3.87 (3H, s), 4.7 ( 1 H, br.s); ¹⁹F NMR: - 66.39 (3F, q, 7.2Hz), -73.28(3F, q, 7.2Hz).

## Claims

1. A method for producing a fluorinated epoxide comprising the step of reacting a fluorinated ethylenically unsaturated compound having the structure:
(R₁)(R₂)C=C(R₃)(R₄)
with a metal hypohalite oxidizing agent in the presence of a phase transfer catalyst to produce the fluorinated epoxide having the structure: 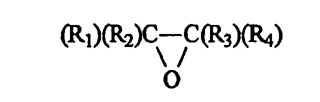 wherein R₁ is selected from the group consisting ofH and OR, where R is C₁-C₁₀ alkyl; R₂ is selected from the group consisting of H, F, C₁-C₁₀ perfluoroalkyl, and X-substituted C₁-C₁₀ alkyl, wherein X is F, Cl, Br, I, OH, or OR; R₃ and R₄ are each independently selected from the group consisting of C₁-C₁₀ perfluoroalkyl, C(R_{f})(R_{f}')OH where R_{f} and R_{f}' are C₁-C₁₀ perfluoroalkyl groups, C₁-C₁₀ perfluoroalkoxy, C₁-C₁₀ carboalkoxy, and hydroxy-substituted C₁-C₁₀ carboalkoxymethyl-substituted C₁-C₄ perfluoroalkyl.

2. A method for producing a fluorinated epoxide according to claim 1 wherein R₁ is selected from the group consisting of H and OR, where R is C₁-C₁₀ alkyl; R₂ is selected from the group consisting ofH and F; R₃ and R₄ are selected from the group consisting of C₁-C₁₀ perfluoroalkyl and C₁-C₁₀ perfluoroalkoxy.

3. The method of claim 1 or claim 2 in which R₂ is H.

## Patentansprüche

1. Verfahren zur Herstellung eines fluorierten Epoxids, umfassend den Schritt der Reaktion einer fluorierten ethylenisch ungesättigten Verbindung, welche die Struktur
(R₁)(R₂)C=C(R₃)(R₄)
aufweist, mit einem Metallhypohalogenit oxidierenden Mittel in Gegenwart eines Phasentransferkatalysators zur Herstellung des fluorierten Epoxids, welches die Struktur 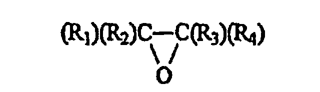 aufweist, worin R₁ aus der Gruppe ausgewählt ist, bestehend aus H und OR, worin R für C₁-C₁₀-Alkyl steht; R₂ aus der Gruppe ausgewählt ist, bestehend aus H, F, C₁-C₁₀-Perfluoralkyl und X-substituiertem C₁-C₁₀-Alkyl, worin X für F, Cl, Br, I, OH oder OR steht; R₃ und R₄ jeweils unabhängig aus der Gruppe ausgewählt sind, bestehend aus C₁-C₁₀-Perfluoralkyl, C(R_{f})(R_{f}')OH, worin R_{f} und R_{f}' C₁-C₁₀-Perfluoralkylgruppen, C₁-C₁₀-Perfluoralkoxy, C₁-C₁₀-Carboalkoxy und Hydroxy-substituiertes C₁-C₁₀-Carboalkoxymethyl-substituiertes C₁-C₄-Perfluoralkyl darstellen.

2. Verfahren zur Herstellung eines fluorierten Epoxids nach Anspruch 1, worin R₁ aus der Gruppe ausgewählt ist, bestehend aus H und OR, worin R für C₁-C₁₀-Alkyl steht; R₂ aus der Gruppe ausgewählt ist, bestehend aus H und F; R₃ und R₄ aus der Gruppe ausgewählt sind, bestehend aus C₁-C₁₀-Perfluoralkyl und C₁-C₁₀-Perfluoralkoxy.

3. Verfahren nach Anspruch 1 oder 2, worin R₂ für H steht.

## Revendications

1. Procédé de production d'un époxyde fluoré comprenant l'étape de mise en réaction d'un compose fluoré à insaturation éthylénique ayant la structure:
(R₁)(R₂)C=C(R₃)(R₄)
avec un agent oxydant hypohalogénite métallique, en présence d'un catalyseur de transfert de phase, pour produire l'époxyde fluoré ayant la structure: 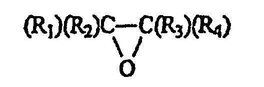 où R₁ est sélectionné parmi le groupe constitué de H, et de OR, où R est un résidu C₁-C₁₀-alkyle; R₂ est sélectionné parmi le groupe constitué des radicaux H, F, C₁-C₁₀ perfluoroalkyle, et C₁-C₁₀-alkyle substitué par X, où X est F, Cl, Br, I, OH ou OR; R₃ et R₄ sont des groupements chacun indépendamment sélectionnés parmi le groupe constitué des radicaux C₁-C₁₀-perfluoroalkyle, C(R_{f})(R_{f}')OH où R_{f} and R_{f}' sont des groupements C₁-C₁₀-perfluoroalkyle, C₁-C₁₀-perfluoroalkoxy, C₁-C₁₀-carboalkoxy, et C₁-C₄-perfluoroalkyle à substitution par un C₁-C₁₀-carboalkoxyméthyle hydroxy-substitué.

2. Procédé de production d'un époxyde fluoré selon la revendication 1, dans lequel R₁ est sélectionné parmi le groupe constitué de H, et de OR, où R est un résidu C₁-C₁₀-alkyle; R₂ est sélectionné parmi le groupe constitué de H et de F; R₃ et R₄ sont sélectionnés parmi le groupe constitué des radicaux C₁-C₁₀ perfluoroalkyle et C₁-C₁₀ perfluoroalkoxy.

3. Procédé selon la revendication 1 ou la revendication 2, dans lequel R₂ est H.
